# EUROPÄISCHE PATENTSCHRIFT

(11) **EP 0 711 756 B1**
(45) Veröffentlichungstag und Bekanntmachung des Hinweises auf die Patenterteilung: **22.07.1998**
(21) Anmeldenummer: 95116888.9
(22) Anmeldetag: 26.10.1995
(51) Int. Cl.: C07D 211/32

(54) **Verfahren zur Herstellung von Benzyl-piperidylmethylindanonen**
Process for the production of benzyl-piperidylmethyl-indanone
Procédé pour la préparation de dérivés de benzyle-piperidylméthyleindanone

(30) Priorität: 08.11.1994 DE 4439822
(43) Veröffentlichungstag der Anmeldung: 15.05.1996
(73) Patentinhaber: BAYER AG, 51368 Leverkusen (DE)
(72) Erfinder: Lensky, Stephen, Dr., D-51515 Kürten (DE)

(56) Entgegenhaltungen:
- EP-A- 0 296 560
- EP-A- 0 535 496
- PATENT ABSTRACTS OF JAPAN vol. 16 no. 180 (C-0935) ,30.April 1992 & JP-A-04 021670 (EISAI CO LTD) 24.Januar 1992,

## Beschreibung

Die vorliegende Erfindung betrifft ein Verfahren zur Herstellung von Benzyl-piperidylmethyl-indanonen. Benzyl-piperidylmethyl-indanone sind Wirkstoffe zur Behandlung von ZNS-Erkrankungen.

In EP 296 560 wurde bereits ein Verfahren zur Synthese von Benzyl-piperidylmethyl-indanonen beschrieben, wobei 1-Benzyl-4-Piperidone in einem vierstufigen Verfahren zum gewünschten Endprodukt umgesetzt werden.

Die Ausbeuten der ersten drei Stufen liegen jeweils unter 65 % d. Th. Darüber hinaus erfordert diese Synthese den Einsatz von Lithium-organischen Verbindungen sowie das Arbeiten unter Inertgasatmosphäre.

In EP 535 496 wird nun ein 3-stufiges Verfahren ausgehend von Pyridin-4-aldehyd und verschiedenen Indanonen beschrieben. Dabei werden die 2-(Pyridin-4-yl-methyl)-ylidenindanone zu Piperidinen hydriert und anschließend hydriert. Dieses Verfahren erfordert aufwendigere Reinigungsverfahren (Chromatogr. + Kristallisation) und führt daher zwangsläufig zu schlechteren Ausbeuten (max. bis zu 29 %).

Es wurde nun ein Verfahren zur Herstellung von Benzyl-piperidylmethyl-indanonen der allgemeinen Formel (I) 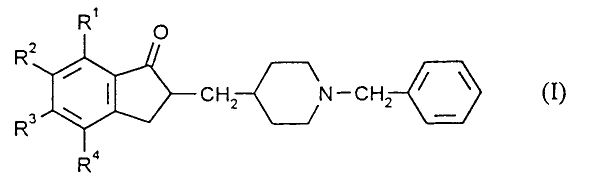 in welcher
- R¹, R², R³ und R⁴: gleich oder verschieden sind und
für Wasserstoff oder
für geradkettiges oder verzweigtes Alkyl, Alkoxy, oder Alkoxycarbonyl mit bis zu 6 Kohlenstoffatomen, Alkyl- oder Dialkyl-(C₁-C₆)-aminocarbonyloxy oder Halogen steht,
gefunden, das dadurch gekennzeichnet ist, daß man
Pyridiniumsalze der Formel (II) 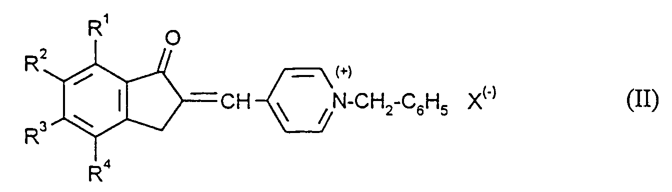 in welcher
- R¹, R², R³ und R⁴: die angegebene Bedeutung haben und
- X^{⊖}: für ein Anion der Reihe Chlorid, Bromid, Iodid, Tosylat, Sulfat steht,
in Gegenwart eines Hydrierkatalysators, bei einer Temperatur von - 20 bis + 120°C, gegebenenfalls in einem inerten Lösemittel mit Wasserstoff hydriert.

Überraschenderweise ist es mit Hilfe des erfindungsgemäßen Verfahrens möglich, die Benzyl-piperidylmethyl-indanone durch direkte Hydrierung bei Normaldruck der Pyridiniumsalze zu erhalten. Bei dieser einstufigen Synthese fallen die Endprodukte in unerwartet hohen Ausbeuten an, insbesondere im Hinblick auf die aus dem Stand der Technik bekannten Verfahren, die schlechte Ausbeuten ergeben und höhere Drücke erfordern.

Bevorzugt werden durch das erfindungsgemäße Verfahren Verbindungen der Formel (I) hergestellt, in welchen R¹ bis R⁴ für Wasserstoff stehen oder R¹ und R⁴ für Wasserstoff stehen und R² und R³, gegebenenfalls unabhängig voneinander, für Methoxy, Methylaminocarbonyloxy, Dimethylaminocarbonyloxy oder Halogen stehen.

Als Lösemittel kommen gegebenenfalls, unabhängig voneinander die üblichen inerten Lösemittel in Frage, die sich unter den Reaktionsbedingungen nicht verändern. Hierzu gehören bevorzugt Ether, wie Diethylether, Dibutylether, Methyl-tert.-butylether, Dioxan oder Tetrahydrofuran, Alkohole wie Methanol, Ethanol, Propanol, Isopropanol oder Butanol, chlorierte Kohlenwasserstoffe wie Dichlormethan, Trichlormethan, Tetrachlormethan oder Dichlorethylen, Aceton, Acetonitril, Dimethylformamid, Dimethylsulfoxid, N-Methylpyrrolidon oder Tetramethylharnstoff, ebenso können Kohlenwasserstoffe wie Hexan, Toluol, Benzol oder Xylol verwendet werden. Darüber hinaus ist es möglich, die genannten Lösemittel als Gemische untereinander sowie als Gemische mit Wasser einzusetzen. Besonders bevorzugt sind Alkohole, insbesondere Methanol oder Ethanol.

Als Hydrierkatalysatoren werden die üblichen, in der organischen Chemie bekannten Hydrierkatalysatoren verwendet. Hierzu gehören insbesondere Platin sowie Platinverbindungen, Palladium, Palladium auf Kohle, Raney-Nickel, Ruthenium sowie Rutheniumverbindungen, gegebenenfalls auf Trägermaterialien wie Aktivkohle oder Siliciumdioxyd. Bevorzugt werden Platin sowie Platinverbindungen und Ruthenium sowie Rutheniumverbindungen. Ganz besonders bevorzugt Platindioxyd (Hydrierkatalysator nach Adams) verwendet.

Die Umsetzung wird im allgemeinen in einem Temperaturbereich von -20 bis +120°C, vorzugsweise von 0 bis +80°C, ganz besonders bevorzugt von +10 bis +35°C durchgeführt.

Die Hydrierung kann bei Normaldruck aber auch bei erhöhtem Druck durchgeführt werden. Im allgemeinen arbeitet man in einem Druckbereich von 1 bis 100 Atmosphären, vorzugsweise von 1 bis 20 Atmosphären Wasserstoffdruck.

Bei der Durchführung der Hydrierung setzt man auf 100 g Pyridiniumsalz der Formel (II) etwa 0,01 bis 200 g, bevorzugt 1 bis 30 g, des zu verwendenden Katalysators ein.

Die Pyridiniumsalze der allgemeinen Formel (II) sind neu und werden hergestellt, indem man
Pyridine der allgemeinen Formel (III) 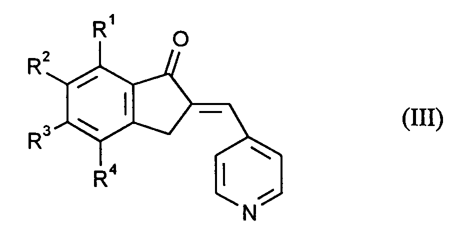 in welcher
- R¹, R², R³ und R⁴: die angegebene Bedeutung haben,
gegebenenfalls in Gegenwart eines inerten Lösemittels mit Verbindungen der allgemeinen Formel (IV) 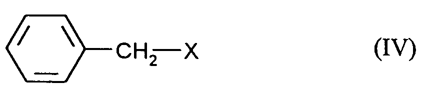 in welcher
- X: für eine übliche Fluchtgruppe aus der Reihe Halogen, bevorzugt Chlor oder Brom, steht,
gegebenenfalls in Gegenwart eines Katalysators umsetzt.

Als inerte Lösemittel werden hierbei bevorzugt die üblichen organischen Lösemittel verwendet, die sich bei der Umsetzung nicht verändern. Hierzu gehören bevorzugt Ether wie Diethylether, Dibutylether, Methyl-tert.-butylether, Dioxan oder Tetrahydrofuran, chlorierte Kohlenwasserstoffe wie Dichlormethan, Trichlormethan oder Tetrachlormethan, Aceton, Acetonitril, Dimethylformamid, Dimethylsulfoxyd, Alkohole wie Methanol, Ethanol, Propanol oder Isopropanol oder N-Methylpyrrolidon, Sulfolan oder Tetramethylharnstoff. Die genannten Lösemittel können auch in Form von Gemischen eingesetzt werden.

Besonders bevorzugt sind Acetonitril, Aceton, Butanon, Dimethylformamid, N-Methylpyrrolidon, Tetramethylharnstoff sowie DMSO.

Als Katalysatoren kommen Alkalimetalliodide und Tosylate in Frage. Bevorzugt sind Kaliumiodid und Natriumiodid.

Die Umsetzung zu den Pyridiniumsalzen kann entweder unter Kühlung, bei Raumtemperatur oder unter Erwärmen durchgeführt werden. Bevorzugt wird bei der Siedetemperatur des eingesetzten Lösemittels oder bei 75-100°C gearbeitet.

Bei der Umsetzung wird im allgemeinen das Pyridin der allgemeinen Formel (III) in der Siedehitze in dem jeweiligen Lösemittel gelöst und die Benzylverbindung der Formel (IV) hinzugefügt. Üblicherweise fällt das Produkt in reiner Form aus oder kann nach dem Abkühlen vollständig zur Kristallisation gebracht werden. Nach Abfiltrieren und Nachwaschen des Niederschlages mit einem inerten Lösemittel kann das Produkt direkt weiterverarbeitet werden.

### Experimenteller Teil

### Beispiel 1

### 2-(Pyridin-4-yl)-methylen-indan-1-on:

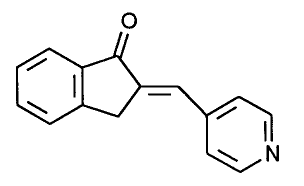

13,2 g Indanon, 15 g Pyridin-4-aldehyd und 19 g p-Toluolsulfonsäure wurden in 250 ml Toluol 5 h am Wasserabscheider unter Rückfluß zum Sieden erhitzt. Der entstandene Niederschlag wurde nach dem Abkühlen abgesaugt und in 10 %iger Natriumkarbonatlösung 30 min bei Raumtemperatur gerührt. Der entstandene, blaßgelbe Niederschlag wurde abgesaugt, mit Wasser gewaschen und an der Luft getrocknet.
Ausbeute: 19,3 g (87 %)

### Beispiel 2

### 5,6-Dimethoxy-2-(pyridin-4-yl)-methylen-indan-1-on:

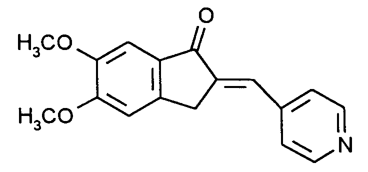

19,2 g 5,6-Dimethoxyindanon, 15 g Pyridin-4-aldehyd und 19 g p-Toluolsulfonsäure wurden in 250 ml Toluol 5 h am Wasserabscheider unter Rückfluß zum Sieden erhitzt. Der entstandene Niederschlag wurde nach dem Abkühlen abgesaugt und in 10 %iger Natriumkarbonatlösung 30 min bei Raumtemperatur gerührt. Der entstandene, blaßgelbe Niederschlag wurde abgesaugt, mit Wasser gewaschen und an der Luft getrocknet.
Ausbeute: 24,4 g (87 %).

### Beispiel 3

### 1-Benzyl-4-(indan-1-on-2-yliden)-methyl-pyridiniumbromid:

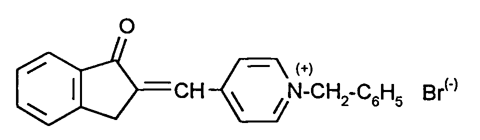

10 g Verbindung des Beispiels 1 wurden in 150 ml siedendem Acetonitril gelöst und in der Wärme 7,5 g Benzylbromid hinzugefügt. Das Gemisch wurde noch weitere 2 h unter Rückfluß zum Sieden erhitzt, wobei das Produkt ausfällte. Man ließ abkühlen, saugte den entstandenen Niederschlag ab, wusch ihn mit Acetonitril und Methyl-tert.-butylether in angegebener Reihenfolge und trocknete an der Luft.
Ausbeute: 14,75 g (83 %)

### Beispiel 4

### 1-Benzyl-4-(5,6-dimethoxyindan-1-on-2-yliden)-methyl-pyridiniumbromid:

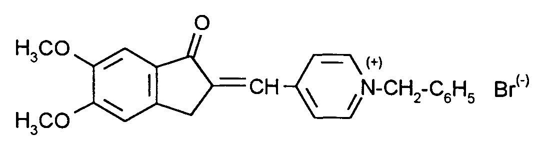

10 g Verbindung des Beispiels 2 wurden in 500 ml siedendem Acetonitril gelöst und in der Wärme 7,5 g Benzylbromid hinzugefügt. Das Gemisch wurde noch weitere 2 h unter Rückfluß zum Sieden erhitzt, wobei das Produkt ausfällte. Man ließ abkühlen, saugte den entstandenen Niederschlag ab, wusch ihn mit Acetonitril und Methyl-tert.-butylether in angegebener Reihenfolge und trocknete an der Luft.
Ausbeute: 14 g (83 %)

### Beispiel 5

### 1-Benzyl-4-(indan-1-on-2-yl)-methyl-piperidin:

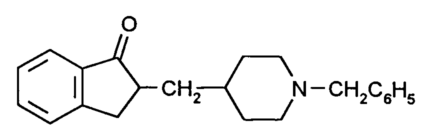

10 g Verbindung des Beispiels 3 und 1 g Platindioxid (Hydrierungskatalysator nach Adams) wurden in 50 ml Methanol suspendiert. Anschließend wurde 24 h bei Normaldruck und bei Raumtemperatur hydriert. Der Katalysator wurde anschließend abfiltriert und das Filtrat eingeengt. Der Rückstand wurde mit 5 % Natriumhydrogenkarbonatlösung aufgenommen und der entstandene Niederschlag abgesaugt, mit Wasser gewaschen und unter Vakuum getrocknet.
Ausbeute: 6,6 g (81 %)

### Beispiel 6

### 1-Benzyl-4-(5,6-dimethoxyindan-1-on-2-yl)-methyl-piperidin:

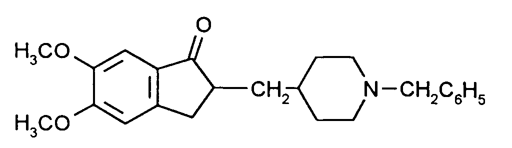

10 g Verbindung des Beispiels 4 und 1 g Platindioxid (Hydrierungskatalysator nach Adams) wurden in 50 ml Methanol suspendiert. Anschließend wurde 24 h bei Normaldruck und bei Raumtemperatur hydriert. Der Katalysator wurde anschließend abfiltriert und das Filtrat eingeengt. Der Rückstand wurde mit 5 % Natriumhydrogenkarbonatlösung aufgenommen, 3 mal mit Dichlormethan extrahiert, getrocknet und eingeengt.
Ausbeute: 6,9 g (81 %)

## Patentansprüche

1. Verfahren zur Herstellung von Benzyl-piperidylmethyl-indanonen der allgemeinen Formel 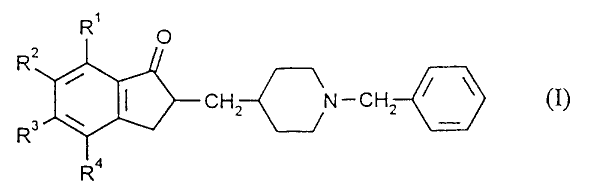 in welcher
R¹, R², R³ und R⁴ gleich oder verschieden sind und
für Wasserstoff oder
für geradkettiges oder verzweigtes Alkyl, Alkoxy oder Alkoxycarbonyl mit bis zu 6 Kohlenstoffatomen, für Alkyl- oder Dialkyl-(C₁-C₆)-aminocarbonyloxy oder Halogen steht,
dadurch gekennzeichnet, daß man
Pyridiniumsalze der Formel (II) 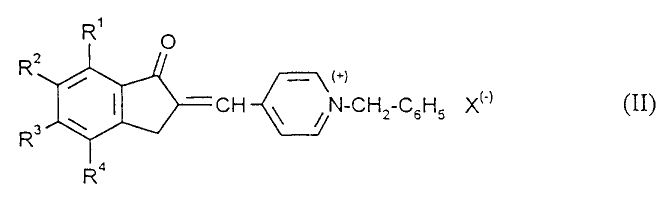 in welcher
R¹, R², R³ und R⁴ die angegebene Bedeutung haben und
X^{⊖} für ein Anion der Reihe Chlorid, Bromid, Iodid, Tosylat oder Sulfat steht,
in Gegenwart eines Hydrierkatalysators, bei einer Temperatur von - 20 bis + 120°C, gegebenenfalls in einem inerten Lösemittel mit Wasserstoff hydriert.

2. Verfahren nach Anspruch 1 zur Herstellung von Verbindungen der allgemeinen Formel (I), worin R¹ bis R⁴ für Wasserstoff stehen.

3. Verfahren nach Anspruch 1 zur Herstellung von Verbindungen der allgemeinen Formel (I), worin R¹ und R⁴ für Wasserstoff stehen und R² und R³ für Methoxy stehen.

4. Verfahren nach Anspruch 1 bis 3, dadurch gekennzeichnet, daß als Hydrierkatalysator Platin oder dessen Verbindungen eingesetzt werden.

5. Verfahren nach Anspruch 1 bis 4, dadurch gekennzeichnet, daß man die Hydrierung mit Wasserstoff bei einem Druck von 1 bis 20 Atmosphären durchführt.

## Claims

1. Process for the preparation of benzyl-piperidylmethyl-indanones of the general formula 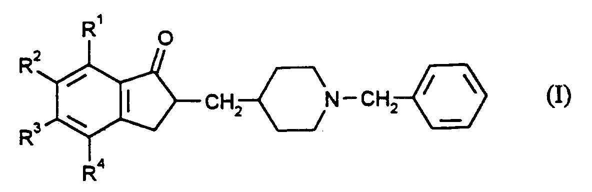 in which
R¹, R², R³ and R⁴ are identical or different and
represent hydrogen or
represent straight-chain or branched alkyl, alkoxy or alkoxycarbonyl having up to 6 carbon atoms, alkyl- or dialkyl-(C₁-C₆)-aminocarbonyloxy or halogen,
characterized in that
pyridinium salts of the formula (II) 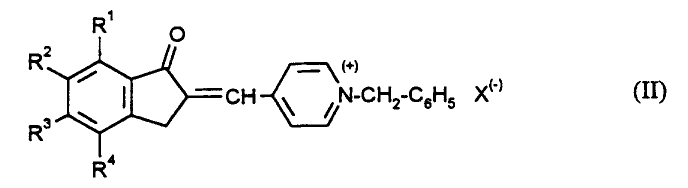 in which
R¹, R², R³ and R⁴ have the meaning indicated and
X^{⊖} represents an anion of the series chloride, bromide, iodide, tosylate, sulphate,
are hydrogenated with hydrogen in the presence of a hydrogenation catalyst at a temperature of -20 to + 120°C, if appropriate in an inert solvent.

2. Process according to Claim 1 for the preparation of compounds of the general formula (I), wherein R¹ to R⁴ represent hydrogen.

3. Process according to Claim 1 for the preparation of compounds of the general formula (I), wherein R¹ and R⁴ represent hydrogen and R² and R³ represent methoxy.

4. Process according to Claims 1 to 3, characterized in that the hydrogenation catalyst employed is platinum or its compounds.

5. Process according to Claims 1 to 4, characterized in that the hydrogenation is carried out at a pressure from 1 to 20 atmospheres using hydrogen.

## Revendications

1. Procédé de production de benzyl-pipéridylméthyl-indanones de formule générale 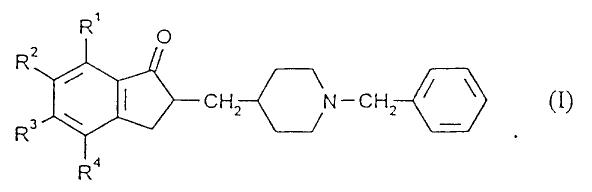 dans laquelle
R¹, R², R³ et R⁴ sont identiques ou différents et représentent
de l'hydrogène ou
un groupe alkyle, alkoxy ou alkoxycarbonyle linéaire ou ramifié ayant jusqu'à 6 atomes de carbone, un groupe alkyl- ou dialkyl-(alkyle en C₁ à C₆)-aminocarbonyloxy ou un halogène,
caractérisé en ce qu'on effectue l'hydrogénation avec de l'hydrogène en présence d'un catalyseur d'hydrogénation à une température de -20 à +120°C, éventuellement dans un solvant inerte, de sels de pyridinium de formule (II) 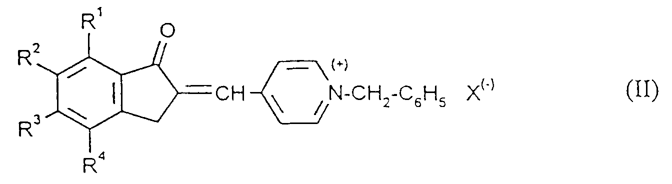 dans laquelle
R¹, R², R³ et R⁴ ont la définition indiquée ci-dessus et
X^{⊖} représente un anion de la série chlorure, bromure, iodure, tosylate ou sulfate.

2. Procédé suivant la revendication 1, pour la production de composés de formule générale (I), dans laquelle R¹ à R⁴ représentent de l'hydrogène.

3. Procédé suivant la revendication 1, pour la production de composés de formule générale (I), dans laquelle R¹ et R⁴ représentent de l'hydrogène et R² et R³ représentent des groupes méthoxy.

4. Procédé suivant les revendications 1 à 3, caractérisé en ce qu'on utilise comme catalyseur d'hydrogénation du platine ou ses composés.

5. Procédé suivant les revendications 1 à 4, caractérisé en ce qu'on conduit l'hydrogénation avec de l'hydrogène à une pression de 1 à 20 atmosphères.
